# EUROPÄISCHE PATENTSCHRIFT

(11) **EP 3 478 072 B1**
(45) Veröffentlichungstag und Bekanntmachung des Hinweises auf die Patenterteilung: **01.09.2021**
(21) Anmeldenummer: 17733449.7
(22) Anmeldetag: 27.06.2017
(51) Int. Cl.: A01N 59/00, A01N 37/16, A01P 1/00, C02F 1/72, A61L 2/22, A61L 11/00

(54) **VERFAHREN UND VORRICHTUNG ZUR OXIDATIVEN BEHANDLUNG VON MATERIAL**
METHOD AND DEVICE FOR THE OXIDATIVE TREATMENT OF MATERIAL
PROCÉDÉ ET DISPOSITIF DE TRAITEMENT DE MATIÈRE PAR OXYDATION

(30) Priorität: 30.06.2016 DE 102016007929
(43) Veröffentlichungstag der Anmeldung: 08.05.2019
(73) Patentinhaber: Nonnenmacher, Klaus, 72070 Tübingen (DE)
(72) Erfinder: Nonnenmacher, Klaus, 72070 Tübingen (DE)
(74) Vertreter: Patentanwaltskanzlei Cartagena
(86) Internationale Anmeldenummer: PCT/EP2017/065845
(87) Internationale Veröffentlichungsnummer: WO 2018/002045

(56) Entgegenhaltungen:
- EP-A1- 2 213 165
- WO-A1-2015/074638
- US-A- 2 857 410
- US-A- 5 816 498
- DATABASE WPI Week 200482 Thomson Scientific, London, GB; AN 2004-826594 & JP 2004 329810 A (KANKI KK) 25 November 2004 (2004-11-25)

## Beschreibung

Die vorliegende Erfindung betrifft ein Verfahren zur oxidativen Behandlung von Material. Das Verfahren ist insbesondere für die Schädlingsbekämpfung bei Pflanzen geeignet.

Im Bereich der Pflanzenkultivierung stellt die Schädlingsbekämpfung einen sehr wichtigen Faktor dar. Insbesondere im Nutzpflanzenanbau können durch verschiedene Schädlinge, wie beispielsweise Insekten, Pilze, Viren und Bakterien, erhebliche Schäden entstehen. Auch im Zierpflanzenanbau ist oftmals eine Schädlingsbekämpfung erforderlich. Es sind vielfältige Bekämpfungsmethoden bekannt, mit denen Schädlinge mehr oderweniger effektiv bekämpft werden können. Neben biologischen Methoden, bei denen beispielsweise natürliche Fressfeinde der Schädlinge eingesetzt werden, wird eine Vielzahl von chemischen Bekämpfungsmitteln eingesetzt. Beispielsweise kommen verschiedene chemische Insektizide, Pestizide, Fungizide und Herbizide in der Praxis zum Einsatz. Die Einbringung von derartigen Substanzen in einen Pflanzenbestand ist allerdings mit erheblichen Problemen behaftet. Es können sich beispielsweise Resistenzen der jeweiligen Schadorganismen gegenüber der Bekämpfungssubstanz entwickeln, so dass eine weitere effektive Bekämpfung nicht mehr möglich ist. Es kann zur Anreicherung der verwendeten Substanzen in den Pflanzen kommen, sodass diese Substanzen in die Nahrungskette gelangen und auch beispielsweise von den Verbrauchern aufgenommen werden. Weiterhin beschränkt sich die Wirkung der verwendeten Substanzen in der Regel nicht nur auf die jeweilige Pflanze bzw. die entsprechenden Schadorganismen, sondern auf das gesamte nähere oder auch weitere Umfeld der Pflanze. Andere Organismen, beispielsweise Nützlinge oder andere Tiere (z. B. Bienen etc.), können durch die Substanzen beeinträchtigt und geschädigt werden. Die verwendeten Substanzen können in das Grundwasser gelangen und damit auch in weiter entfernten Gebieten schädliche Wirkungen entfalten.

Die deutsche Patentschrift DE 197 28 609 C2 offenbart die Herstellung von Triglycerid-Sorbinsäure-Pantothenyl-Acetalperoxiden durch Behandlung einer heterogenen Mischung von flüssigen ungesättigten Triglyceriden (Pflanzenölen), flüssigem Pantothenyl-Alkohol, festen feinteiligen oder flüssigen Polyethylenglykolen und fester feinteiliger Sorbinsäure mit ozonhaltigem Sauerstoff, wobei die hergestellte Substanz in Form einer wässrigen Emulsion zur Bekämpfung von pathogenen Pilzen oder Bakterien bei einer äußerlichen oder innerlichen Anwendung beim Menschen vorgesehen ist. Die deutsche Offenlegungsschrift DE 199 32 570 A1 offenbart die Verwendung dieser Substanz, die als mit Ozon angereichertes Oliven- und Rizinusöl beschrieben wird, für eine antimikrobielle Behandlung im Zahnbereich, wobei in Bezug auf die Herstellung dieser Substanz auf die zuvor genannten Patentschrift verwiesen wird. Die Substanz wird direkt auf oder im Zahnfleisch appliziert.

Aus der WO 2015/074638 A1 sind ein Verfahren und eine Vorrichtung bekannt, die zur oxidativen Behandlung einer Flüssigphase und/oder einer Gasphase und/oder einer Festphase vorgesehen sind. Hierbei wird für die Behandlung Ozon und wenigstens eine Komponente, die durch die Ozonisierung von wenigstens einem Olefin bereitgestellt wird, verwendet. Das Verfahren und die Vorrichtung können beispielsweise für eine Abwasserbehandlung aber auch zur Bekämpfung von Pflanzenschädlingen eingesetzt werden.

Aus der US 2 857 410 A ist ein Verfahren zur Bildung von Ozoniden aus ungesättigten Fettsäuren bekannt.

Die JP 2004-329810 A betrifft ein Deodorant, das ein Ölsäure-basiertes Ozonid in einer Sprühvorrichtung umfasst. Das Ozonid wird trocken hergestellt. Zur Applikation wird es in einer Ethanol-Wasser-Mischung gelöst. Für die ozonisierte Ölsäure werden keine POZ-Bereiche genannt.

Aus der EP 2 213165 A1 ist eine Zusammensetzung bekannt, die sich als antiseptisches, antimikrobielles, desodorierendes, insektizides oder repellierendes Mittel eignet. Die Zusammensetzung enthält ein ozonisiertes Tensid. Das ozonisierte Tensid ist durch die Zugabe von Ozon zu einem Tensid mit wenigstens einer olefinischen Doppelbindung in einem hydrophoben Rest erhältlich. Bei dem Tensid kann es sich um ein anionisches Tensid handeln, beispielsweise um eine ungesättigte Fettsäure.

Aus der US 5 816 498 A ist ein Verfahren bekannt, bei dem Pflanzen mit einer Ozon-Wasser-Mischung besprüht werden. Ozonide oder Hydroperoxide sind auch hier nicht erwähnt.

Die vorliegende Erfindungstelltsich gegenüber der Problematik bei der herkömmlichen Verwendung von Schädlingsbekämpfungsmitteln die Aufgabe, ein verbessertes und breit einsetzbares Verfahren zur oxidativen Behandlung von Material bereitzustellen, das auch und mit besonderem Vorteil bei der Schädlingsbekämpfung bei Pflanzen einsetzbar ist. Diese Aufgabe wird durch ein Verfahren gelöst, wie es sich aus dem Anspruch 1 ergibt. Vorteilhafte Ausführungen dieses Verfahrens sind Gegenstand der abhängigen Ansprüche.

Die Erfindung stellt ein Verfahren zur oxidativen Behandlungvon Material bereit, bei dem organische Ozonide und organische Hydroperoxide auf das Material gesprüht werden. Vorzugsweise wird zur Bereitstellungeines sprühbaren Ansatzes eine Mischung der organischen Ozonide und organischen Hydroperoxide mit Wasser bereitgestellt. Prinzipiell ist es auch möglich, dass die Ozonide und Hydroperoxide direkt versprüht werden, wobei je nach Viskosität des Ozonid-/Hydroperoxidmaterials beispielsweise entsprechende hohe Drücke oder eine Erhitzung des Ozonid-/Hydroperoxidmaterials vorgesehen sein kann. Erfindungsgemäß werden die Ozonide und Hydroperoxide auf das Material gesprüht, wobei sich auf der Materialoberfläche ein mehr oder weniger fettiger Film absetzt. Die Ozonide und Hydroperoxide bzw. der Film setzen auf dem Material und in der unmittelbaren Umgebung des Materials verschiedene oxidative Prozesse in Gang, die sehr vorteilhafte Auswirkungen haben. Insbesondere kann dieses Verfahren zur Schädlingsbekämpfung bei Pflanzen eingesetzt werden, indem der Sprühansatz auf die Pflanzen oder in den Bereich der Pflanzen gesprüht wird. Hierbei werden die mit Schädlingen befallenen Pflanzen zweckmäßigerweise mit dem Sprühansatz benetzt. Das Besprühen hat den besonderen Vorteil, dass die Ozonid-/Hydroperoxidmasse direkt auf die Pflanzen gelangt und damit unmittelbar in den Pflanzen ihre Wirkung in besonders effektiver Weise entfalten kann. Weiterhin bewirkt das Versprühen eine Nebelbildung, sodass die Pflanzen (oder allgemein das zu behandelnde Material) von der hierbei entstehenden desinfizierenden und schädlingsabtötenden Atmosphäre umgeben werden/wird. Für das Besprühen kann die Ozonid-/Hydoperoxidmasse sehr sparsam verwendet werden. Überraschende Versuchsergebnisse des Erfinders haben gezeigt, dass die Behandlung der Pflanzen mit organischen Ozoniden und organischen Hydroperoxiden in sehr effektiver Weise eine Bekämpfung der Schädlinge erzielt, wobei die Pflanze selbst sich sehr schnell von dem Schädlingsbefall erholt und sich im Weiteren besonders gut entwickelt. Das erfindungsgemäße Verfahren hat weiterhin den besonderen Vorteil, dass sehr unterschiedliche Schädlinge wirksam bekämpft werden können, da die verwendeten Ozonide und Hydroperoxide unspezifisch auf verschiedene Pilze, Bakterien, Viren oder Schadinsekten wirken, ohne dass die Pflanze selbst dabei angegriffen wird. Darüber hinaus stellt die Behandlung mit Ozoniden und Hydroperoxiden keine Belastung für die Umwelt dar, da sich diese Substanzen im Zuge der Anwendung selbst vollständig abbauen und dabei zusätzlich einen reinigenden Effekt insbesondere in der Luft im Sinne einer Luftentkeimung bewirken. Weiterhin üben die verwendeten organischen Ozonide und Hydroperoxide in gewisser Weise eine Depotwirkung aus, die sich über den gesamten Zeitraum des oxidativ wirkenden Abbaus dieser Substanzen erstreckt, sodass die Behandlung mit einer verhältnismäßig geringen Dosis dieser Substanzen erfolgen kann. Da die Wirkung der Ozonide und organischen Hydroperoxide auf einer verhältnismäßig unspezifischen oxidativen Wirkung beruht, können sich Resistenzen gegenüber dieser Behandlung nicht entwickeln. Wenn die Ozonide und Hydroperoxide erfindungsgemäß verwendet werden, kann auf andere beispielsweise chemische Behandlungsmethoden (z.B. Spritzen von Insektiziden) verzichtet werden. Somit ergibt sich ein erheblicher Vorteil für die Umwelt, da das erfindungsgemäße Verfahren keine Umweltbelastung darstellt. Ein besonderer Vorteil des erfindungsgemäßen Ansatzes ist, dass sich auch das Ozonid oder die Ozonidmasse selbst von sich aus abbaut. Das Ozonid zerstörtsich gewissermaßen selbst durch das von ihm selbst produzierte Wasserstoffperoxid. Die Zwischenprodukte sind Aldehyde, Carbonsäuren und Wasser, bis zuletzt nur noch Kohlendioxid und Wasser übrig bleibt. Das Gleiche gilt für die Hydroperoxidmasse.

Bei den organischen Ozoniden und organischen Hydroperoxiden handelt es sich insbesondere um Substanzen, die durch eine Behandlung von ungesättigten Fettsäuren mit ozonhaltigem Sauerstoff herstellbar sind. Im Folgenden werden die organischen Ozonide und Hydroperoxide auch allgemein als Ozonid-/Hydroperoxidmasse (oder Ozonide/Hydroperoxide) bezeichnet. Für die Herstellung der Ozonide und Hydroperoxide wird zweckmäßigerweise gasförmiger und mit Ozon angereicherter Sauerstoff eingesetzt, der vorzugsweise in eine Reaktionsmischung mit den Fettsäuren bei intensiver Durchmischung eingeleitet wird.

Erfindungsgemäß wird der Reaktionsmischung Wasser in einem Anteil von bis zu 45 Gew.-% (bezogen auf das Gesamtgewicht der eingesetzten Fettsäuren und des insgesamt zugesetzten Wassers) zugesetzt. In weiter bevorzugten Ausführungsformen werden der Reaktionsmischungzwischen 0,5 und 20 Gew.-%, besonders bevorzugt zwischen 0,5 und 10 Gew.-%, insbesondere zwischen 0,5 und 5 Gew.-%, Wasser zugesetzt (ebenfalls bezogen auf das Gesamtgewicht der eingesetzten Fettsäuren und des insgesamt zugesetzten Wassers). Das Zusetzen des Wassers kann in einem Schritt oder kontinuierlich über einen längeren Zeitraum erfolgen.

Besonders bevorzugt ist der Anteil an zugesetztem Wasser stöchiometrisch auf die Menge der in den Fettsäuren enthaltenen Doppelbindungen abgestimmt. Demzufolge sollte idealerweise maximal 1 mol H₂O/mol CC-Doppelbindungen zugesetzt werden. Das Wasser kann in einer solchen Menge während der Ozonisierung vollständig verbraucht werden.

Produkt der Ozonisierung ist bei einem vollständigen Verbrauch des Wassers in Folge einer unterstöchiometrischen Zugabe eine trockene Ozonid-/Hydroperoxidmasse. Gemäß bevorzugten Ausführungsformen der Erfindung wird genau dies angestrebt, da die trockene Ozonid-/Hydroperoxidmasse gut lagerbar und transportierbar ist. Hierzu später noch mehr.

Besonders bevorzugt werden den Fettsäuren je mol CC-Doppelbindungen zwischen 0,01 und 1 mol Wasser, bevorzugt von 0,1 mol bis 0,9 mol Wasser, weiter bevorzugt von 0,1 mol bis 0,75 mol Wasser, besonders bevorzugt von 0,1 mol bis 0,7 mol Wasser, zugesetzt. Innerhalb der genannten Bereiche sind Wasseranteile von 0,3 mol bis 0,9 mol, bevorzugt von 0,3 mol bis 0,75 mol, insbesondere von 0,5 mol bis 0,75 mol Wasser je mol CC-Doppelbindungen für einige Anwendungen weiter bevorzugt.

Bei der Herstellung der organischen Ozonide und organischen Hydroperoxide ist es prinzipiell möglich, feuchten oder trockenen ozonhaltigen gasförmigen Sauerstoff zu verwenden. Die Verwendung von trockenem ozonhaltigem Sauerstoff ist in der Regel bevorzugt.

Sofern die die Fettsäuren enthaltende Reaktionsmischung auch Wasser enthält, insbesondere in einem Anteil innerhalb der genannten bevorzugten Bereiche, können sich bei der Reaktion mit dem Ozon des trockenen Sauerstoffs ebenfalls organische Hydroperoxide bilden. Das Wasser ist also Reaktionspartner. Grundsätzlich kann das Wasser den Fettsäuren direktzugesetzt werden. Eine Zuführung des Wassers ist aber auch in Form des feuchten ozonhaltigen gasförmigen Sauerstoffs möglich.

In besonders bevorzugter Weise wird reine Ölsäure als Fettsäure für die Herstellung der organischen Ozonide und organischen Hydroperoxide verwendet, da sich die hieraus herstellbaren Substanzen als besonders effektiv für die erfindungsgemäßen Zwecke erwiesen haben.

Bei der Umsetzung der Fettsäuren mit dem Ozon können neben den gewünschten Produkten auch Nebenprodukte, beispielsweise gebildet durch einen Zerfall bereits gebildeter Hydroperoxide und Ozonide oder aber durch sonstige Nebenreaktionen, entstehen. In aller Regel ist es nicht erforderlich, diese Nebenprodukte in einem separaten Schritt abzutrennen. Sie finden sich dann später in der sprühbaren Mischung wieder, wie auch gegebenenfalls nicht umgesetzte Fettsäuren.

In einer besonders bevorzugten Ausführungsform des Verfahrens werden organische Ozonide verwendet, die durch die Behandlung der ungesättigten Fettsäuren mit trockenem ozonhaltigem Sauerstoff herstellbar sind, wobei insgesamt der Herstellungsprozess der Ozonide vorzugsweise unter aprotischen Bedingungen durchgeführt wird. Gemäß dem von Rudolf Criegee beschriebenen Reaktionsmechanismus werden hierbei neben der Bildung von verschiedenen Zwitterionen ein Primärozonid (Molozonid) und ein Sekundärozonid (1,2,4-Trioxolan) gebildet (Lit.: Von Sonntag, Chemistry of ozone in water and waste water treatment, Seite 85, ISBN 9781843393139). Das bei dieser Reaktion letztendlich gebildete Sekundärozonid wird auch als Criegee-Ozonid bezeichnet und ist für die Zwecke der vorliegenden Erfindung besonders bevorzugt.

Die Verwendung der trocken bzw. aprotisch hergestellten organischen Ozonide für die Zwecke der Erfindung hat gegenüber der Verwendung von organischen Hydroperoxiden für bestimmte Anwendungen verschiedene Vorteile. So sind die im Folgenden noch näher erläuterten Peroxidzahlen (POZ) von Ozoniden im Allgemeinen höher als die Peroxidzahlen von Hydroperoxiden. Damit ist auch die Wirksamkeit der Ozonide in dem erfindungsgemäßen Verfahren im Allgemeinen höher. Die trocken hergestellte Ozonidform wartet dabei gewissermaßen dringlich auf Wasser, um mit diesem zum desinfizierend wirkenden Wasserstoffperoxid zu reagieren. Das feucht hergestellte organische Hydroperoxid hatsich das Wasser gewissermaßen schon einverleibt, sodass sich das Wasserstoffperoxid bereits in der Hydroperoxidsubstanz befindet. Hinzu kommt, dass die Lagerfähigkeit des organischen Hydroperoxids sehr begrenzt ist. Die trocken gelagerte Ozonidform hingegen ist lange haltbar und stabil, wobei die Reaktivität in der erfindungsgemäßen Anwendung erhalten bleibt.

Wichtig hierbei ist, dass kein Wasser in die Ozonidsubstanz vor der Anwendung (während der Lagerung oder während eines Transports, etwa zu einem Applikationsort) eindiffundiert, weshalb die Ozonidsubstanz vor ihrer erfindungsgemäßen Anwendung entsprechend wasserdiffusionsdicht verpackt sein sollte. Für die Verpackung bzw. Lagerung eignet sich beispielsweise Glas.

Kurzum, in einigen besonders bevorzugten Ausführungsformen des erfindungsgemäßen Verfahrens werden die organischen Ozonide und organischen Hydroperoxide vor ihrer Anwendung, d.h. bevor sie in einer Mischung mit Wasser für das Sprühen bereitgestellt und auf das Material gesprüht werden, trocken gelagert, insbesondere wasserdiffusionsdicht verpackt gelagert. Dies gilt insbesondere für die in Folge der beschriebenen unterstöchiometrischen Zugabe von Wasser erhaltene trockene Ozonid-/Hydroperoxidmasse. Bei nicht trockener Lagerung würden sich die Ozonide in der Ozonid-/Hydroperoxidmasse zersetzen.

Als Transport- und/oder Lagerbehältnisse für die organischen Ozonide und organischen Hydroperoxide eignen sich insbesondere Behälter aus Glas oder aus wasserdiffusionsdichten Verbundfolien. Geeignete Verbundfolien weisen beispielsweise eine dünne Lage aus einem Metall als Diffusionssperre auf.

Zweckmäßigerweise weist das verwendete organische Ozonid und organische Hydroperoxid einen relativ hohen Dampfdruck auf, da in verschiedenen Ausgestaltungen des erfindungsgemäßen Ansatzes auch die sich aus der Ozonid-/Hydroperoxidmasse entwickelnde Gasphase bzw. die sich entwickelnden Dämpfe die desinfizierende Wirkung entfalten. Diese Dämpfe können im Prinzip alle Bereiche des zu behandelnden Materials erreichen. Zusammen mit der Feuchtigkeit bzw. dem Wasserdampf, der gegebenenfalls von dem Material, zum Beispiel von der Pflanze, abgegeben wird, kommt es zur Ausbildung von desinfizierend wirkendem Wasserstoffperoxid, sodass sich eine desinfizierende und schädlingsabtötende Atmosphäre im unmittelbaren Umfeld des behandelten Materials einstellt.

Der Dampfdruck der Ozonid-/Hydroperoxidmasse hängt unter anderem von der Peroxidzahl (POZ) der Masse ab. Die POZ stellt dabei eine Kennzahl für den Gehalt an peroxidischen bzw. reaktiven Sauerstoffgruppen der jeweiligen Substanz (Fett oder Öl) dar. Die POZ wird gemäß DIN-Normen mittels Titration, beispielsweise mittels Iodometrie gemäß DIN EN ISO 3960:2017, bestimmt und wird in "MilliÄquivalent Sauerstoff je Kilogramm Substanz (meq/kg)" angegeben. Für die Zwecke der Erfindung werden organische Ozonide und organische Hydroperoxide mit POZ in einem Bereich zwischen 1000 - 2500 meq/kg. Dies gilt auch wenn reine Ölsäure oder eine zu mehr als 50 % aus Ölsäure bestehende Zusammensetzung als Fettsäure für die Herstellung der organischen Ozonide und organischen Hydroperoxide verwendet wird.

Die POZ lässt sich durch die Bedingungen bei der Ozonid- und Hydroperoxid-Herstellung steuern. So lässt sich bei der Herstellung der organischen Ozonide und Hydroperoxide die POZ gezielt auf die genannten bevorzugten Bereiche einstellen. Beispielsweise erhöht die Reinheit bzw. Qualität des verwendeten ozonhaltigen Sauerstoffgases die POZ des gebildeten Produktes. Weiterhin besteht ein logarithmischer Zusammenhang zwischen der Dauer der Ozonisierung und dem POZ-Wert des ozonidhaltigen Produktes, wobei die POZ mit zunehmender Dauer ansteigt und je nach Reaktionsbedingungen beispielsweise bei 6 h eine Sättigung zeigt. In der Praxis hat sich für die Herstellung der Ozonid-/Hydroperoxidmasse beispielsweise eine Ozonisierungsdauer von 4 h bei einem Batch-Verfahren bewährt.

Insbesondere ist es auch durch den Zusatz von Wasser zu der oben erwähnten Reaktionsmischung möglich, das Verhältnis der Menge entstehenden Ozonids zur Menge entstehenden Hydroperoxids und damit auch die Peroxidzahl (POZ) gezielt zu beeinflussen. Grundsätzlich gilt, dass ein geringerer Wasserzusatz in der Reaktionsmischung mit einem höheren Anteil an entstehendem Ozonid verknüpft ist. Und je höher der Anteil des entstehenden Ozonids ist, desto höher ist in der Regel auch die POZ.

So wird zur Einstellung der POZ der organischen Ozonide und organischen Hydroperoxide auf den genannten Bereich zwischen 1000 und 2500 meq/kg der Wasserzusatz in der genannten Reaktionsmischung in der Regel auf einen Wert unterhalb einer der genannten Obergrenzen von 45 Gew.-% eingestellt, insbesondere bei Verwendung reiner Ölsäure oder einer zu mehr als 50 %, besonders bevorzugt zu mehr als 65 %, aus Ölsäure bestehenden Zusammensetzung als Fettsäure für die Herstellung der organischen Ozonide und organischen Hydroperoxide.

Der Wasser enthaltenden Reaktionsmischung wird zur Herstellung der organischen Ozonide und organischen Hydroperoxide bevorzugt trockener ozonhaltiger Sauerstoff zugesetzt. Unter trockenem ozonhaltigem Sauerstoff soll hierbei Sauerstoff verstanden werden, der Wasser allenfalls in einer derart geringen Menge enthält, dass hierdurch das erwähnte Verhältnis der Menge entstehenden Ozonids zur Menge entstehenden Hydroperoxids nicht beeinflusst wird.

In einigen bevorzugten Ausführungsformen werden bei der Herstellung der organischen Ozonide und organischen Hydroperoxide mehr als 60 % Gew.-%, bevorzugt mehr als 80 Gew.-%, insbesondere mehr als 90 Gew.-%, der eingesetzten ungesättigten Fettsäuren zu den organischen Ozoniden verarbeitet. Das genaue Verhältnis kann insbesondere durch den Wasserzusatz in der Reaktionsmischung eingestellt werden.

Umgekehrt bedeutet das, dass in diesen Ausführungsformen weniger als 10 Gew.-% der eingesetzten Fettsäuren zu den Hydroperoxiden verarbeitet werden. In besonders bevorzugten Ausführungsformen kann dieser Anteil sogar noch deutlich tiefer liegen, insbesondere < 5 Gew.-% betragen, besonders bevorzugt nahe bei Null liegen.

Einige Versuche deuten darauf hin, dass die erwähnte Depotwirkung verstärkt wird, je mehr Ozonid bei der Herstellung anteilig gebildet wird.

Zur Bereitstellungeiner sprühbaren Mischung werden die organischen Ozonide und organischen Hydroperoxide bevorzugt in einer Mischung mit Wasser für das Sprühen bereitgestellt.

In einer Mischung mit Wasser reagieren die organischen Ozonide, beispielsweise die Ozonide in der in Folge der beschriebenen unterstöchiometrischen Zugabe von Wasser erhaltenen trockenen Ozonid-/Hydroperoxidmasse, mit dem Wasser, wobei es zur Bildung von Wasserstoffperoxid kommt. Faktisch enthält die Mischung, in der die organischen Ozonide und organischen Hydroperoxide mit dem Wasser für das Sprühen bereitgestellt werden, somit immer auch Wasserstoffperoxid. Zur Funktion des Wasserstoffperoxids bei dem erfindungsgemäßen Behandlungsverfahren finden sich in den folgenden Absätzen noch einige Ausführungen.

Wie bereits ausgeführt, eignet sich das erfindungsgemäße Verfahren in besonderer Weise zur Schädlingsbekämpfung bei Pflanzen. Vorzugsweise wird eine Mischung der Ozonide/Hydroperoxide mit Wasser auf die Pflanze oder in den Pflanzenbestand gesprüht. Durch das Besprühen gelangt die Mischung gut verteilt direkt auf die Pflanzen und setzt sich als Film beispielsweise auf den Blättern und dem Spross ab, sodass die Mischung ihre Wirkung direkt entfalten kann.

Grundsätzlich gilt, dass die erzielte Konzentration von Wasserstoffperoxid abhängig ist vom Ansatz und von der Art der Ozonid/Hydroperoxid-Grundsubstanz sowie von den Bedingungen bei der Herstellung der sprühbaren Mischung.

Zur Herstellung der Mischung für das Sprühen können die organischen Ozonide und organischen Hydroperoxide mit Wasser versetzt werden, das auf eine Temperatur im Bereich von 20 °C bis 50 °C, insbesondere von 30 °C bis 40°C, besonders bevorzugt auf etwa 30 °C, temperiert wurde. Alternativ kann man die organischen Ozonide und organischen Hydroperoxide auch mit Wasser versetzen und die entstehende Mischung auf die genannten Temperaturen erwärmen.

Durch die Temperierung des Wassers bzw. die Erwärmung der Mischung kann die Mischbarkeit des Wassers mit den organischen Ozoniden und den organischen Hydroperoxiden verbessert werden. Insbesondere kann aber auch die erwähnte Bildung von Wasserstoffperoxid beschleunigt werden.

Das erfindungsgemäße Verfahren kann prophylaktisch oder bei erkanntem Schädlingsbefall eingesetzt werden. Ein besonderer Vorteil des Verfahrens ist, dass durch die sich im Zuge der erfindungsgemäßen Behandlung einstellende oxidative Atmosphäre, die vor allem durch sich bildendes Wasserstoffperoxid und OH-Radikale bewirkt wird, verhältnismäßig unspezifisch auf eine sehr große Bandbreite von Schädlingen wirkt. Eine genaue Analyse des jeweiligen Schädlingsbefalls vorab ist daher nicht erforderlich. Darüber hinaus eignet sich das erfindungsgemäße Verfahren auch aus diesem Grund in besonderer Weise für eine prophylaktische Pflanzenbehandlung zur Vermeidung eines Schädlingsbefalls.

Für das Besprühen kann die Ozonid-/Hydoperoxidmasse sehr sparsam verwendet werden. Beispielsweise kann auf 1 m³ Wasser circa 0,001 kg bis ca. 1 kg der Masse gegeben werden. 10 l dieser Mischung ist im Allgemeinen ausreichend für ca. 1 ha Pflanzenbestand. Das Versprühen kann mit üblicher Sprühtechnik und üblichen Sprühmaschinen erfolgen. Für den Anwender ergibt sich damit auch der besondere Vorteil, dass er bereits vorhandene Maschinen für die Zwecke des erfindungsgemäßen Verfahrens nutzen kann.

Es kann besonders bevorzugt sein, eine für das Sprühen bereitgestellte Mischung der organischen Ozonide und Hydroperoxide mit Wasser unmittelbar auf das jeweilige Material zu sprühen. Es kann aber auch bevorzugt sein, die Mischung beim oder unmittelbar vor dem Sprühen zu verdünnen, insbesondere durch Vermengung mit Wasser.

Besonders bevorzugt erfolgt das Sprühen auf das Material mit Hilfe einer Düse. Bei der Düse kann es sich um eine Einstoff-Druckdüse handeln, der die Mischung der organischen Ozonide und organischen Hydroperoxide mit Wasser unter Druck zugeführt wird und die Energie zu ihrer Zerstäubung in sich trägt. Alternativ kann die Mischung in der Düse auch unter Druck mit einem sekundären Medium, insbesondere mit Luft oder einer Flüssigkeit wie Wasser, vermischt werden. Das sekundäre Medium kann der Düse unter Druck zugeführt werden und als Treibmedium dienen, mit dessen Hilfe die Mischung zerstäubt wird.

Das Besprühen kann beispielsweise im Freiland erfolgen. Hierbei ergibt sich ein zusätzlicher Vorteil, da durch die Behandlung mit Ozonid/Hydroperoxid gleichzeitig eine luftreinigende Wirkung erzielt wird. Die erfindungsgemäße Behandlung hat insbesondere bei einer Anwendung im Freiland den besonderen Vorteil, dass das aufgebrachte Ozonid/Hydroperoxid durch Regen oder andere Umwelteinflüsse in Wesentlichen nicht von den Pflanzen abgewaschen wird. Daher ist es in der Regel ausreichend, die Behandlung zu Beginn der Wachstumsperiode und später noch einmal etwa in der Mitte der Wachstumsperiode durchzuführen. Falls ein hoher Schädlingsdruck auftritt, kann die Behandlung gegebenenfalls häufiger durchgeführt werden. Diese nicht so häufig erforderliche Durchführung der erfindungsgemäßen Behandlung ist ein weiterer besonderer Vorteil gegenüber herkömmlichen Schädlingsbekämpfungsverfahren. Beispielsweise müssen herkömmliche Pestizide im Weinbau in der Regel innerhalb von etwa 7 Monaten 5 - 7 mal gespritzt werden.

In einer anderen Ausgestaltung des erfindungsgemäßen Verfahrens wird die Behandlung des Materials und insbesondere der Pflanzen in einem im Wesentlichen abgeschlossenen Raum durchgeführt. Dies hat den besonderen Vorteil, dass sich durch den abgeschlossenen Raum die Konzentration der Ozonid-/Hydroperoxiddämpfe erhöht und damit eine besonders effektive Schädlingsbekämpfung oder anderweitige oxidative Behandlung möglich ist. Fürdie Realisierung dieser Ausführungsform kann der Pflanzenbestand nach dem Besprühen mit der Ozonid/Hydroperoxid-Mischung mit einer Folie oder Ähnlichem abgedeckt werden. Weiterhin ist es auch möglich, dass die Behandlung der Pflanzen oder allgemein des Materials von Beginn an in einem Behälter erfolgt. Befallene Pflanzen können beispielsweise zusammen mit dem jeweiligen Pflanztopf in einen entsprechenden Behälter eingesetzt und behandelt werden. Weiterhin ist es auch möglich, dass entsprechende Behälter auf die zu behandelnden Pflanzen aufgesetzt oder übergestülpt werden oder Pflanzen mit einer Folie oder ähnlichem abgedeckt werden. Prinzipiell ist es auch möglich, das erfindungsgemäße Verfahren in größeren Behältereinheiten, die mehr als eine Pflanze fassen, durchzuführen, beispielsweise in Gewächshäusern, Zelten oder Ähnlichem. Eine feuchte Atmosphäre in derartigen Räumen verstärkt im Allgemeinen den Effekt des erfindungsgemäßen Verfahrens. Nach der schädlingsbekämpfenden Behandlung können die Pflanzen aus dem Behälter entnommen werden oder die Abdeckung kann gegebenenfalls entfernt werden, bevor die Pflanzen unter üblichen Bedingungen weiter kultiviert werden.

Bei den zu behandelnden Pflanzen kann es sich um die verschiedensten Pflanzen handeln, insbesondere um Nutzpflanzen oder Zierpflanzen. Es können beispielsweise Gehölze behandelt werden. In ganz besonderer Weise eignet sich das erfindungsgemäße Verfahren für die Schädlingsbekämpfung bei Weinreben, die sehr anfällig für verschiedene Arten von Schädlingen sind. Beispielsweise kann die Kirschessigfliege in sehr effektiver Weise mit dem erfindungsgemäßen Verfahren bekämpft werden. Die Kirschessigfliege sticht die Frucht an und produziert damit ein Loch, das die Kirschessigfliege für die Eiablage nutzt. Die sich bei dem erfindungsgemäßen Verfahren entwickelnden Dämpfe dringen in das Loch ein, das sich nach dem Anstechen durch die Kirschessigfliege nicht mehr verschließt. In dem Loch befindet sich genügend Wasser, sodass durch das sich im Zuge der Behandlung entwickelnde Wasserstoffperoxid und andere oxidative Substanzen das Ei abgetötet wird. Auch Schimmelpilze (z. B. Botrytis cinerea - Blauschimmel) oder andere Schadorganismen, die im Weinbau zu erheblichen Problemen führen können, können erfindungsgemäß wirksam und umweltschonend behandelt werden.

Wenn das Ozonid/Hydroperoxid auf die Pflanzen aufgesprüht wird, entstehen durch die von der Pflanze abgegebene Feuchtigkeit im unmittelbaren Umfeld der Pflanze Ozonid/Hydroperoxid-Dämpfe. Diese Dämpfe wirken auch auf die benachbarten Pflanzen, sodass verhältnismäßig geringe Abstände zwischen den einzelnen Pflanzen für das erfindungsgemäße Verfahren vorteilhaft sein können. Bezogen auf den Weinbau kann es daher unter Umständen vorteilhaft sein, den Abstand zwischen den Rebstockreihen verhältnismäßig klein zu halten.

Das erfindungsgemäße Verfahren kann weiterhin in effektiver Weise für eine Behandlungvon Pfropfen oder von gepfropften Pflanzen eingesetzt werden. Im Weinbau beispielsweise werden die Pfropfen herkömmlicherweise etwa im November in einem Desinfektionsbad mit Chlor gewaschen und dann bis etwa Februar in einem Raum gelagert, bevor die Pfropfen an einen Winzer ausgeliefert werden. Der Winzer führt dann einen Schnitt an dem Rebstock aus und setzt den Pfropfen ein. Während der Lagerung des Pfropfens und während und nach dem Vorgang des Pfropfens selbst bestehen Infektionsrisiken, insbesondere durch Schimmelpilze. Die erfindungsgemäße Behandlung der Pfropfen und der gepfropften Pflanzen mit Ozonid/Hydroperoxid stellt demgegenüber eine besonders umweltfreundliche Lösung dar. Hierbei werden zunächst die Pfropfen selbst mit Ozonid/Hydroperoxid erfindungsgemäß behandelt, gegebenenfalls noch unterstützt durch den Zusatz von Ozon. Das aufgesprühte Ozonid-/Hydroperoxid verbleibt als Film auch während der nachfolgenden Lagerung auf dem Pfropfen, sodass eine Langzeitwirkung erzielt wird. Alternativ oder zusätzlich kann ein Bad in einer Ozonid/Hydroperoxid-Mischung durchgeführt werden. Während des Lagerzeitraums können die Pfropfen wiederholt ein oder mehrfach mit den Ozoniden/Hydroperoxiden besprüht werden, gegebenenfalls wieder kombiniert mit Ozon. Während des Pfropfvorgangs werden die Schnittstelle und der Pfropfen direkt mit den Ozoniden/Hydroperoxiden besprüht. Alternativ oder zusätzlich kann die Schnittstelle und der Pfropfen direkt mit einer Ozonid-/Hydroperoxidmasse (z.B. Ozonidcreme oder - paste) bestrichen werden.

Die sprühende Verwendung der Ozonide/Hydroperoxide kann auch mit Vorteil für andere Anwendungen, bei denen eine oxidative Behandlung durchgeführt werden soll, eingesetzt werden. Vor allem eignet sich das erfindungsgemäße Verfahren aufgrund der desinfizierenden Wirkung für eine desinfizierende Behandlung von Abfällen. Hierbei wird die Ozonid-/Hydroperoxidmasse vorzugsweise mit Wasser gemischt und auf die Abfälle gesprüht. Prinzipiell ist es auch möglich, die Ozonid-/Hydroperoxidmasse ohne weitere Verdünnungzu versprühen, beispielsweise mit entsprechend hohen Drücken oder bei erhöhter Temperatur. Die desinfizierenden Wirkmechanismen bei der Behandlung der Abfälle beruhen ebenfalls auf den beschriebenen oxidativen Prozessen. Beispielsweise kann das Verfahren für die Behandlung von Hygieneabfällen, beispielsweise zur Behandlung von gebrauchten Babywindeln oder Ähnlichem, eingesetzt werden. Weiterhin eignet sich das erfindungsgemäße Verfahren in besonderer Weise auch für die Zwischenlagerung von Krankenhausabfällen, die verschiedene organische Anteile, z.B. Blut und/oder Urin, enthalten können und die mit einer erheblichen Keimbelastung versehen sein können, die eine Infektionsquelle für die damit in Berührung kommenden Personen darstellen können. Krankenhausabfälle werden oftmals vor ihrem Abtransport zu einer Entsorgungsanlage geschreddert. Auch dieses geschredderte Material kann während der Lagerung und/oder des Transportes erfindungsgemäß behandelt werden, wodurch das infektiöse Gefahrenrisiko, das mit einer Keimbelastung des Materials einhergeht, verringert wird.

Eine zur Durchführung des erfindungsgemäßen Verfahrens geeignete Vorrichtung umfasst wenigsten eine Düse zum Versprühen von organischen Ozoniden und organischen Hydroperoxiden. Die Vorrichtung umfasst weiterhin wenigstens eine Konditioniereinheit zur Bereitstellung einer sprühbaren Mischung aus organischen Ozoniden und organischen Hydroperoxiden mit Wasser und/oder einen Tank mit einer fertig angesetzten Sprühmischung sowie eine Pump- oder Kompressoreinheit. Bezüglich weiterer Merkmale insbesondere der Mischung, die mit dieser Vorrichtung auf das zu behandelnde Material aufgebracht werden kann, beispielsweise auf Pflanzen oder auf Abfälle, wird auf die obige Beschreibung verwiesen.

**Der Tank kann nachfüllbarsein und eine wiederverschließbare Füllöffnung aufweist, sodass eine "frische" sprühfertige Ozonid**-/Hydroperoxidmischung vom Anwender bei Bedarf nachgefüllt werden kann. Es kann auch vorgesehen sein, dass der Tank für den Anwender nicht wiederbefüllbar ist und also keine zugängliche Füllöffnung aufweist. In dieser Ausgestaltung wird der Tank einmalig befüllt und dann für die Anwendung bereitgestellt.

Die Vorrichtung kann beispielsweise in Form eines Steckknopfes mit einer Düse, einem Tank und beispielsweise mit einem Blasebalg oder einer anderen Sprühvorrichtung ausgestaltet sein. Der Tank wird mit einer sprühfertigen Mischung befüllt. Eine Konditioniereinheit ist in diesem Fall nicht vorgesehen. Die sprühfertige Mischung wird also außerhalb vorbereitet. Die Vorrichtung kann gegebenenfalls zusammen mit einer geeigneten Dichtung in eine Öffnung eines Behälters, der das zu behandelnde Material enthält, eingesteckt werden. Durch Betätigung des Blasebalgs wird die Ozonid/Hydroperoxid-Mischung in den Behälter gesprüht und benetzt das dort vorhandene Material.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Zeichnungen zeigen:
- Figur 1: die Darstellung einer Vorrichtung zum Sprühen von Ozonid/Hydroperoxid; und
- Figur 2A-C: die schematische Darstellung von Ausführungsmöglichkeiten einer Sprühvorrichtung; und
- Fig. 3: den Einfluss des Wasserzusatzes in einer Reaktionsmischung mit Ölsäure auf die Peroxidzahl (POZ); und
- Fig. 4: die H₂O₂-Entwicklung verschiedener Ozonide in 0,1 %-iger wässriger Lösung.

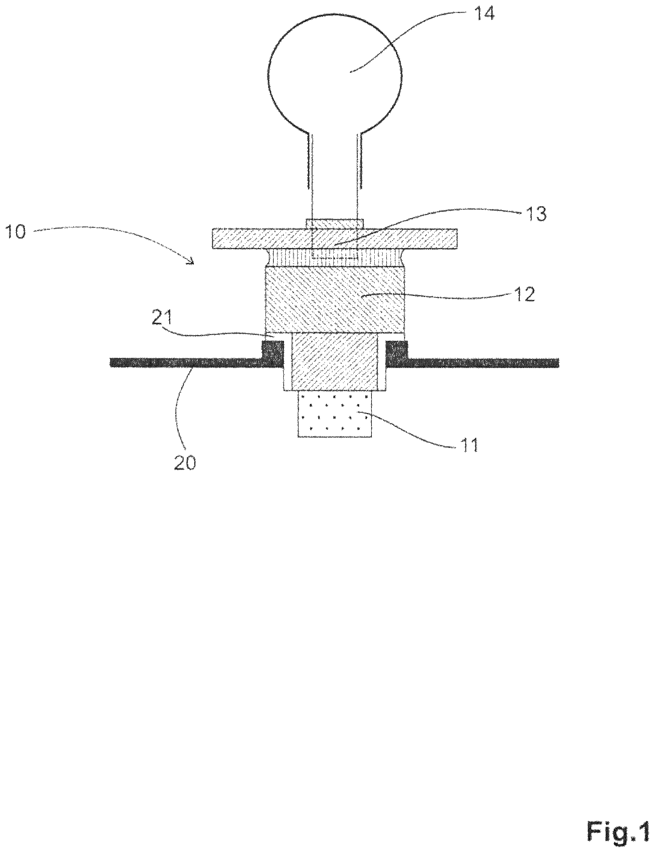

Fig. 1 illustriert ein Beispiel für eine Einrichtung oder Vorrichtung 10 zum Sprühen der Ozonid-/Hydroperoxidmasse bzw. einer geeigneten sprühbaren Ozonid-/Hydroperoxid-Mischung. Die Vorrichtung 10 ist in steckknopfartiger Weise ausgebildet und kann beispielsweise für die Schädlingsbekämpfung bei einer Pflanze, die sich in einem Behälter befindet, oder für die Behandlung von Müll in einem Abfallbehälter verwendet werden. Hierfür wird die Vorrichtung 10 in eine entsprechende Öffnung der Behälterwandung 20 oder eines Deckels des Behälters, gegebenenfalls zusammen mit einer Dichtung 21, eingesetzt. Die Vorrichtung 10 umfassteine oder mehrere Düsen 11, die in das Innere des Behälters (hier nicht näher dargestellt) ragen. Die Ozonid-/Hydroperoxidmasse ist in einem integrierten Vorratstank 12, gegebenenfalls in verdünnter Form, vorzugsweise in einer Mischung mit Wasser (Sprühmischung), bevorratet. DerVorratstank 12 kann übereine Füllöffnung 13 befüllt werden. DerVorratstank 13 kann gegebenenfalls wiederholt mit frischer Sprühmischung vom Anwender befüllt werden, sodass die Vorrichtung 10 für einen längeren Behandlungszeitraum oder zum Versprühen von größeren Mengen der Sprühmischung verwendet werden kann. Die Vorrichtung 10 umfasst weiterhin einen Blasebalg 14, der auf den Vorratstank 12 aufgesetzt ist. Die Düsen 11 werden über den Blasebalg 14 betätigt, wobei Gas oder Luft zwangsweise durch den Blasebalg 14 dem Vorratstank 12 zugeführt wird und die Sprühmischungdurch die Düsenöffnungen der Düsen 11 drückt. Hierbei wird die Sprühmischung zerstäubt und gelangt auf die zu behandelnden Pflanzen oder gegebenenfalls auf anderes Material. Alternativ kann statt eines Blasebalgs beispielsweise ein Spritzpistolenmechanismus oder Ähnliches vorgesehen sein. Durch das Versprühen setzt sich die Mischungzum einen als Film auf dem zu behandelnden Material ab. Zum anderen kommt es zu einer Nebelbildung durch die versprühten Tröpfchen. Zusätzlich kann es zu einer sekundären Aerosolbildung durch die ablaufenden chemischen Reaktionen der freigesetzten Ozonid/Hydroperoxid-Dämpfe mit vorhandenem Wasser kommen, das beispielsweise von den zu behandelnden Pflanzen als Feuchtigkeit abgegeben wird.

Für die Herstellung der organischen Ozonide und organischen Hydroperoxide werden ungesättigte Fettsäuren mit ozonhaltigem und vorzugsweise gasförmigem Sauerstoff behandelt. In besonders bevorzugter Weise werden hierfür Pflanzenöle eingesetzt. Beispielsweise ist Ölsäure besonders geeignet, wobei die Ölsäure aus einer pflanzlichen oder auch aus einer tierischen Quelle stammen kann. Die Ozonisierung der Fettsäure erfolgt vorzugsweise mit trockenem sauerstoffhaltigem Gas, vorzugsweise mit trockenem medizinischem oder industriellem Sauerstoff in einer Reinheit von > 99,5 oder > **99,9% (wasserfrei), der mit Ozon angereichert wurde. Die "Trockenheit" des Sauerstoffs lässt sich über** den Taupunkt definieren. Beisp**ielsweise ist "trockener" Sauerstoff mit einem Taupunkt im Bereich** von 200 Kelvin für die erfindungsgemäßen Zwecke geeignet. Der Ozonisierungsprozess wird vorzugsweise in einem geschlossenen Gefäß mit Kühlung durchgeführt, wobei in dem Gefäß die zu ozonisierende Fettsäure vorgelegt wird und bei intensiver Durchmischung der ozonhaltige trockene Sauerstoff eingeleitet wird. Vorteilhafterweise wird der Prozess unter vollständigem Ausschluss von Wasser durchgeführt, wobei zweckmäßigerweise wasserdampfundurchlässige Gefäße, Leitungen usw. (diffusionsdicht für gasförmiges Wasser) verwendet werden. Der Prozess kann beispielsweise im Batch-Verfahren bei einer Ozonisierungszeit von etwa 4 h durchgeführt. werden. Dabei bildet sich an der C-C-Doppelbindung der Fettsäure ein Ozonid. Ein weiteres Reaktionsprodukt kann sich in Gegenwart von Wasser mit einer funktionellen HOO-**Peroxidgruppe am α**-ständigen C-Atom bilden. Quelle für das Wasser kann beispielsweise das zugeführte gegebenenfalls feuchte, mit Ozon angereicherte Sauerstoffgas sein oder die Umgebungsatmosphäre beim Ozonisieren der Fettsäure oder eine gegebenenfalls vorgesehene Wasserzugabe zu der Fettsäure bzw. zu dem Reaktionsansatz vor dem Beginn der Ozonisierung. Je nach Herstellungsbedingungen bildet sich ein öliges bis pastöses Produkt (Ozonid-/Hydroperoxidmasse). Dieses Addukt ist beständig und lagerfähig. Der Dampfdruck steigt mit dem Grad der Ozonisierung und ist von den Ausgangssubstanzen und den Reaktionsbedingungen abhängig. Für die Fettsäure-Komponente bei der Ozonid-/Hydroperoxidherstellung können prinzipiell auch Mischungen verschiedener Fettsäuren und insbesondere Mischungen verschiedener Pflanzenöle, beispielsweise verschiedene ungesättigte Triglyceride, gegebenenfalls mit weiteren Zusätzen, verwendet werden.

Besonders bevorzugtes Ausgangsprodukt für die Herstellung der organischen Ozonide ist Ölsäure. Die Ölsäure kann beispielsweise aus Olivenöl stammen, das zu etwa 70 % aus Ölsäure besteht. Vorzugsweise erfolgt die Ozonisierung der reinen Ölsäure mit trockenem, ozonhaltigem Sauerstoff unter aprotischen Bedingungen. Als Produkt dieses Prozesses wird eine ölige bis pastöse Ozonidmasse erhalten, die frei von störenden Nebenprodukten, wie z.B. Aldehyde, Ketone und Wasserstoffperoxid, ist. Die Ozonidmasse ist stabil und über einen langen Zeitraum lagerfähig. Für eine Stabilisierung dieser Ozonidmasse sind keine weiteren Zusätze, keine Extraktion und keine Reduktionsmittel erforderlich. Der Dampfdruck der ozonisierten Ölsäure kann durch den Grad der Ozonisierung angepasst werden. Der Dampfdruck ist bei höherer POZ größer. Weiterhin zeichnet sich eine Ozonidmasse mit einer höheren POZ im Allgemeinen durch einen stärkeren Geruch aus.

Zur Herstellung einer sprühbaren Mischung wird die Ozonid/Hydroperoxid vorzugsweise mit Wasser gemischt. Um die Mischbarkeit zu verbessern, kann das Wasser z. B. auf etwa 30 °C temperiert werden. Die Durchmischung kann in einfacher Weise mechanisch, beispielsweise durch Rühren, erfolgen.

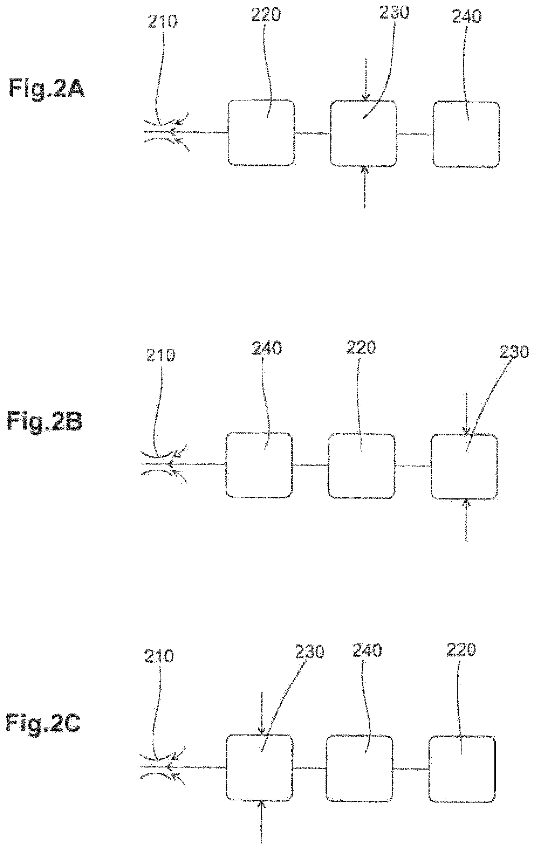

Fig. 2A-C illustriert in schematischer Weise drei Möglichkeiten zur Realisierung einer Sprühvorrichtung für das erfindungsgemäße Verfahren. Die in den Fig. 2A-C gezeigten Ausführungsmöglichkeiten umfassen jeweils eine Düse 210, einen Tank 220, eine Konditionierstufe 230 und eine Pump- oder Kompressoreinheit 240.

Die Düse 210 ist zum Sprühen oder Zerstäuben der Ozonide/Hydroperoxide (sprühfertige Mischung) vorgesehen, wobei das Sprühen oderZerstäuben mit oder ohne einem sekundären Medium (Luft oder Flüssigkeit, hier angedeutet durch Pfeile), das beispielsweise durch Druck und/oder die Geschwindigkeit beim Sprühvorgang im Bereich der Düse 210 eingesaugt werden kann, durchgeführt werden kann. Hierbei kann es gegebenenfalls vorteilhaft sein, wenn als Sekundärmedium beispielsweise Ozon in Form von mit Ozon angereicherter Luft oder von ozonisiertem Wasser eingesogen und mit versprüht wird. Insbesondere durch eine Kombination von Ozon mit dem Ozonid/Hydroperoxid kann unter Umständen eine besonders vorteilhafte Wirkung bei der Schädlingsbekämpfung oder bei einer Abfallbehandlung oder bei einer anderen oxidativen Behandlung durch die bei dieser Kombination ablaufenden oxidativen Prozesse erreicht werden.

In der Konditionierstufe 230 erfolgt die Bereitstellung der sprühfertigen Mischung aus der Ozonid-/Hydroperoxidmasse und Wasser (angedeutet durch vertikale Pfeile). Die Konditionierstufe 230 kann apparativ mit einer Mischeinrichtung ausgeführt und in die Sprühvorrichtung integriert sein. Andererseits ist es auch möglich, die Konditionierstufe 230 als manuelle Vorstufe zu realisieren, wobei beispielsweise das Wasser mit der Ozonid-/Hydroperoxidmasse mit oder ohne Temperierung verrührt wird. Für eine Temperierung kann beispielsweise warmes Wasser (z. B. 30 °C) verwendet werden, wodurch sich die Mischbarkeit bei der Bereitstellung der sprühfertigen Mischung verbessern lässt. Die sprühfertige Mischung kann gegebenenfalls in dem Tank 220 zwischengelagert werden und/oder direkt zum Versprühen oder Zerstäuben verwendet werden.

Statt einer separaten Konditionierstufe 230 in der Sprühvorrichtung kann beispielsweise auch eine Flasche oder ein anderer Behälter mit entsprechend konfektioniertem Inhalt verwendet werden, wobei die Konditionierung ausgelagert ist und gewissermaßen nur ein Tank 220 für die fertige Sprühmischung vorgesehen ist. Diese Ausgestaltung ist im Prinzip bei der Sprühvorrichtung realisiert, die in Fig. 1 in beispielhafter Weise gezeigt ist, wobei hier als Pumpe 240 ein Blasebalg 14 verwendet wird. Die Konditionierstufe 230 ist als manuelle Vorstufe ausgelagert und die angerührte Sprühmischung wird in den Tank 12 eingefüllt und dann versprüht.

Die einzelnen Elemente 210, 220, 230 und 240 der Sprühvorrichtung können in unterschiedlicher Weise miteinander kombiniert werden. Wie in Fig. 2A gezeigt, kann sich der Tank 220 direkt hinter der Düse 210, gegebenenfalls mit einem entsprechenden Leitungsanschluss, befinden. An den Tank 220 kann sich die Konditionierstufe 230 anschließen, die den Tank 220 mit einer sprühfertigen Mischung versorgt. Hinter der Konditionierstufe 230 befindet sich die Pumpe 240 (Medium Flüssigkeit) oder der Kompressor 240 (Medium Luft). In Fig. 2B ist eine Ausführungsform gezeigt, bei der sich hinter der Düse 210 die Kompressor- oder Pumpeinheit240 befindet. Daran schließtsich der Tank 220 an. Hinter dem Tank 220 befindet sich die Konditionierstufe 230. Fig. 2C zeigt eine Ausführungsform, bei der sich hinter der Düse 210 die Konditionierstufe 230 befindet. Daran schließt sich die Kompressor-oder Pumpeinheit 240 an, wobei sich hinter der Kompressor- oder Pumpeinheit 240 der Tank 220 befindet.

Weiterhin ist es möglich, dass die Konditionierstufe 230 und der Tank 220 miteinander kombiniert werden. Weiterhin ist es möglich, dass beispielsweise ein kleiner Behälter mit der Ozonid-/Hydroperoxidmasse in die Sprühvorrichtung integriert wird und diese Masse batchweise oder kontinuierlich in die Konditionierstufe 230 eingeleitet wird, um batchweise oder kontinuierlich die sprühfertige Mischung bereitstellen zu können, wobei die sprühfertige Mischung direkt versprüht oder gegebenenfalls in dem Tank 220 zwischengelagert werden kann.

Für das Sprühen im Freiland kann ein üblicher Sprühapparat eingesetzt werden. Wenn der Apparat beispielsweise einen Vorratsbehälter mit einem Fassungsvolumen von 5 l hat, werden auf 5 l Wasser beispielsweise 50 g der Ozonid- und Hydroperoxidmasse gegeben. Das Wasser kann temperiert werden. Der Ansatz kann manuell gemischt (z. B. gerührt) werden. In anderen Anwendungsbeispielen werden beispielsweise 50 ml oder 15 ml einer fluiden Ozonid-/Hydroperoxidmasse auf 5 l Wasser (30 °C) gegeben und gemischt, wobei eine messbare Konzentration von 10 mg H₂O₂/l bzw. 5 mg H₂O₂/l erreichbar ist. Anschließend wird der Sprühvorgang vorzugsweise unmittelbar gestartet. Bei derVerwendung von organischen Hydroperoxiden, die im Allgemeinen eine niedrigere POZ als Ozonide aufweisen, entwickelt sich sehr schnell Wasserstoffperoxid, also bereits beim Mischen. Bei der Verwendung von aprotisch hergestellten organischen Ozoniden dauert die Entwicklung von Wasserstoffperoxid Stunden bis Tage. Diese längerfristige Entwicklung von Wasserstoffperoxid und damit die Langzeitwirkung der organischen Ozonide ist für die Anwendung in der Schädlingsbekämpfung oder beispielsweise auch für eine Müllbehandlung besonders vorteilhaft.

### Einfluss des Wasserzusatzes in einer Reaktionsmischung mit Ölsäure auf die Peroxidzahl (POZ)

Wie bereits erwähnt, kann in bevorzugten Ausführungsformen die Peroxidzahl (POZ) durch den Zusatz von Wasser gezielt beeinflusst werden. In Versuchen wurden Reaktionsmischungen mit unterschiedlichen Wasserzusätzen zur Herstellung von Ozoniden/Hydroperoxiden verwendet und bei ansonsten identischen Reaktionsbedingungen über 4 Stunden mit ozonhaltigem trockenem Sauerstoff umgesetzt. Als ungesättigte Fettsäure kam Ölsäure zum Einsatz.

Wie der folgenden Tabelle zu entnehmen ist, wurden die größten POZ-Werte erhalten, wenn der Wasserzusatz bei Null (POZ = 1840 meq/kg) lag. Je höher der Wasserzusatz gewähltwurde, desto geringere POZ-Werte wurden erhalten. Bei einem Wasserzusatz von 96,5 Gew.-% lagen die erhaltenen POZ-Werte nur noch bei 68.

| Ozonidprodukt | Wasseranteil / Vol.-% | Wasseranteil / Gew.-% | POZ / meq kg⁻¹ |
|---|---|---|---|
| Z250/0 | 0,0 | 0,0 | 1840 |
| Z250/1 | 0,4 | 0,4 | 1790 |
| Z250/10 | 3,8 | 4,2 | 1660 |
| Z200/50 | 20,0 | 21,6 | 1440 |
| Z150/100 | 40,0 | 42,3 | 1050 |
| Z10/250 | 96,2 | 96,5 | 68 |

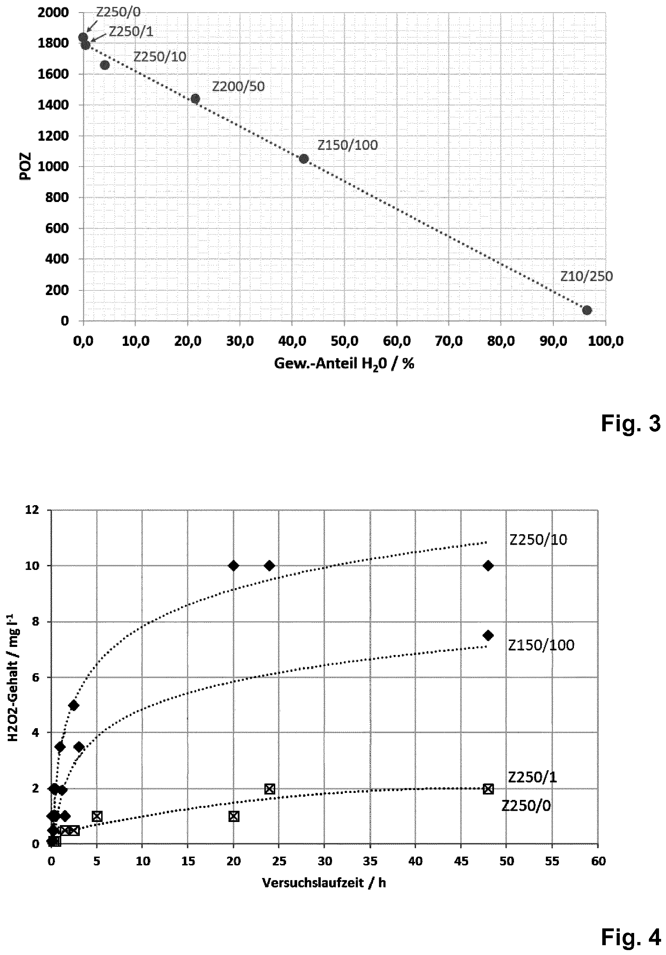

Der Zusammenhang zwischen dem Wasserzusatz der Reaktionsmischung und der POZ ist über den gesamten Variationsbereich hinweg im Wesentlichen linear. Dies geht anschaulich aus Fig. 3 hervor, in der die in der Tabelle gelisteten POZ-Werte gegen den Gewichtsanteil Wasser der jeweiligen Reaktionsmischungen aufgetragen wurden.

Bei der Herstellung sprühbarer Mischungen aus den in der Tabelle aufgeführten Ozonidprodukten wurde allerdings festgestellt, dass eine hohe POZ nicht zwingend gleichbedeutend mit einer hohen Aktivität sein muss.

Bei oxidativen Behandlungen, insbesondere im Rahmen der Schädlingsbekämpfung bei Pflanzen, ist die oben erwähnte Bildung von Wasserstoffperoxid durch Hydrolyse von Ozonid wichtig. Insbesondere zu diesem Zweck werden die organischen Ozonide und organischen Hydroperoxide gemäß dem erfindungsgemäßen Verfahren ja auch in einer Mischung mit Wasser für das Sprühen bereitgestellt.

Mit den in der Tabelle aufgeführten Ozonidprodukten wurden nun Versuche durchgeführt, um die Produkte hinsichtlich ihrer Eignung zur Bildung von Wasserstoffperoxid zu vergleichen. Hierzu wurde in Becherglas-Versuchen die H₂O₂-Bildung beim Einsatz der verschiedenen Ozonidprodukte in verdünnten, wässrigen Lösungen verfolgt. Als Indikator dienten H₂O₂-Teststäbchen (Firma Merck). Die Ergebnisse dieser Untersuchungen sind in Fig. 4 zusammengefasst.

Überraschenderweise führten ausgerechnet die Ozonidprodukte mit den höchsten gemessenen POZ-Werten (Z 250/0 und / 250/1) zu relativ niedrigen Wasserstoffperoxidkonzentrationen. Die höchste Konzentration an Wasserstoffperoxid wurde im Fall von Z250/10 beobachtet. Es scheint also, dass sich eine nicht zu geringe Menge an Wasser günstig auf die Konzentration an Wasserstoffperoxid in der sprühbaren Mischung mit Wasser auswirken kann. Zu hoch sollte die Konzentration allerdings nicht sein. Bei höheren Wasserkonzentrationen sank die Konzentration an Wasserstoffperoxid wieder.

## Patentansprüche

1. Verfahren zur oxidativen Behandlung von Material, **dadurch gekennzeichnet, dass** organische Ozonide und organische Hydroperoxide auf das Material gesprüht werden, wobei
• die organischen Ozonide und organischen Hydroperoxide durch eine Behandlung von ungesättigten Fettsäuren mit ozonhaltigem Sauerstoff hergestellt werden,
• bei der Herstellung der organischen Ozonide und der organischen Hydroperoxide der ozonhaltige Sauerstoff mit den ungesättigten Fettsäuren eine Reaktionsmischung bildet und der Reaktionsmischung Wasser in einem Anteil von bis zu 45 Gewichtsprozent zugesetzt wird, und
• bei der Herstellung die Peroxidzahl (POZ) der organischen Ozonide und organischen Hydroperoxide in der Reaktionsmischung auf einen Wert zwischen 1000 und 2500 meq/kg eingestellt wird
• und die so hergestellten organischen Ozonide und organischen Hydroperoxide in einer Mischung mit Wasser für das Sprühen bereitgestellt werden.

2. Verfahren nach Anspruch 1, **dadurch gekennzeichnet, dass** die Reaktionsmischung einen Wasserzusatz von 0,5 bis 20 Gewichtsprozent, besonders bevorzugt von 0,5 bis 10 Gew.-%, insbesondere von 0,5 bis 5 Gew.-%, aufweist.

3. Verfahren nach einem der vorhergehenden Ansprüche, **dadurch gekennzeichnet, dass** die organischen Ozonide durch Behandlung von Ölsäure mit trockenem ozonhaltigen Sauerstoff herstellbarsind.

4. Verfahren nach einem der vorhergehenden Ansprüche, **dadurch gekennzeichnet, dass** bei der Herstellung der organischen Ozonide und organischen Hydroperoxide mehr als 60 % Gew.-%, bevorzugt mehr als 80 Gew.-%, insbesondere mehr als 90 Gew.-%, der ungesättigten Fettsäuren zu den organischen Ozoniden verarbeitet werden.

5. Verfahren nach einem der vorhergehenden Ansprüche, **dadurch gekennzeichnet, dass** die organischen Ozonide und organischen Hydroperoxide vor ihrer Anwendung auf das Material wasserdiffusionsdicht verpackt gelagert werden.

6. Verfahren nach einem der vorhergehenden Ansprüche, wobei zur Herstellung der Mischung für das Sprühen die organischen Ozonide und organischen Hydroperoxide mit Wasser versetzt werden, das auf eine Temperatur im Bereich von 20 °C bis 50 °C, insbesondere von 30 °C bis 40 °C, besonders bevorzugt auf etwa 30 °C, temperiert wurde.

7. Verfahren nach einem der vorhergehenden Ansprüche, wobei zur Herstellung der Mischung für das Sprühen die organischen Ozonide und organischen Hydroperoxide mit Wasser versetzt werden, und die entstehende Mischung auf eine Temperatur zwischen 20 °C und 50 °C, bevorzugt auf etwa 30 °C, erwärmt wird.

8. Verfahren nach einem der vorhergehenden Ansprüche, wobei das Sprühen auf das Material mit Hilfe einer Düse erfolgt, in der die Mischung unter Druck mit einem sekundären Medium, insbesondere mit Luft oder einer Flüssigkeit wie Wasser, vermischt wird.

9. Verfahren nach einem der vorhergehenden Ansprüche, **dadurch gekennzeichnet, dass** das Verfahren im Freiland oder in einem im Wesentlichen abgeschlossenen Raum erfolgt.

10. Verfahren nach einem der vorhergehenden Ansprüche, **dadurch gekennzeichnet, dass** das Material in einen Behälter eingebracht wird und dass das Verfahren in dem Behälter durchgeführt wird.

11. Verfahren nach einem der vorhergehenden Ansprüche, **dadurch gekennzeichnet, dass** das Material Pflanzen umfasst, wobei das Verfahren zur Schädlingsbekämpfung bei Pflanzen eingesetzt wird, wobei die Pflanzen Gehölze oder Weinreben oder Pfropfen oder gepfropfte Pflanzen sind.

12. Verfahren nach einem der Ansprüche 1 bis 10, **dadurch gekennzeichnet, dass** das Material Abfälle umfasst, wobei das Verfahren zur desinfizierenden Behandlung der Abfälle eingesetzt wird.

## Claims

1. Method for the oxidative treatment of material, **characterized in that** organic ozonides and organic hydroperoxides are sprayed onto the material, where
• the organic ozonides and organic hydroperoxides are prepared by treatment of unsaturated fatty acids with ozone-containing oxygen,
• during the preparation of the organic ozonides and the organic hydroperoxides, the ozone-containing oxygen forms a reaction mixture with the unsaturated fatty acids, and water in a fraction of up to 45 per cent by weight is added to the reaction mixture, and
• during the preparation, the peroxide number (PON) of the organic ozonides and organic hydroperoxides in the reaction mixture is adjusted to a value of between 1000 and 2500 meq/kg,
• and the organic ozonides and organic hydroperoxides thus prepared are provided in a mixture with water for spraying.

2. Method according to Claim 1, **characterized in that** the reaction mixture comprises from 0.5 to 20 per cent by weight of added water, more preferably from 0.5 to 10 wt%, more particularly from 0.5 to 5 wt%.

3. Method according to either of the preceding claims, **characterized in that** the organic ozonides are preparable by treatment of oleic acid with dry, ozone-containing oxygen.

4. Method according to any of the preceding claims, **characterized in that** during the preparation of the organic ozonides and organic hydroperoxides, more than 60 wt%, preferably more than 80 wt%, more particularly more than 90 wt% of the unsaturated fatty acids are processed to the organic ozonides.

5. Method according to any of the preceding claims, **characterized in that** before being applied to the material, the organic ozonides and organic hydroperoxides are stored in packaging impervious to water diffusion.

6. Method according to any of the preceding claims, where the mixture for spraying is prepared by admixing the organic ozonides and organic hydroperoxides with water conditioned to a temperature with a range of 20°C to 50°C, more particularly from 30°C to 40°C, very preferably to about 30°C.

7. Method according to any of the preceding claims, where the mixture for spraying is prepared by admixing the organic ozonides and organic hydroperoxides with water, and the mixture formed is warmed to a temperature of between 20°C and 50°C, preferably to about 30°C.

8. Method according to any of the preceding claims, where the mixture is sprayed onto the material by means of a nozzle in which the mixture is mixed under pressure with a secondary medium, more particularly with air or a liquid such as water.

9. Method according to any of the preceding claims, **characterized in that** the method takes place in the open air or in a substantially closed-off space.

10. Method according to any of the preceding claims, **characterized in that** the material is introduced into a container and that the method is carried out in the container.

11. Method according to any of the preceding claims, **characterized in that** the material comprises plants, the method being used for pest control in plants, and the plants being woody plants or vines or plugs or grafted plants.

12. Method according to any of Claims 1 to 10, **characterized in that** the material comprises wastes, the method being used for the disinfectant treatment of the wastes.

## Revendications

1. Procédé de traitement oxydatif d'un matériau, **caractérisé en ce que** des ozonures organiques et des hydroperoxydes organiques sont pulvérisés sur le matériau, dans lequel
• les ozonures organiques et les hydroperoxydes organiques sont fabriqués par un traitement d'acides gras insaturés avec de l'oxygène contenant de l'ozone,
• lors de la fabrication des ozonures organiques et des hydroperoxydes organiques, l'oxygène contenant de l'azote forme avec les acides gras insaturés un mélange réactionnel, et on ajoute au mélange réactionnel de l'eau selon une proportion allant jusqu'à 45 pour cent en poids, et
• lors de la fabrication, on ajuste l'indice de peroxyde (IP) des ozonures organiques et des hydroperoxydes organiques dans le mélange réactionnel à une valeur entre 1 000 et 2 500 méq/kg
• et les ozonures organiques et hydroperoxydes organiques ainsi fabriqués sont mis à disposition dans un mélange avec de l'eau pour la pulvérisation.

2. Procédé selon la revendication 1, **caractérisé en ce que** le mélange réactionnel présente une addition d'eau de 0,5 à 20 pour cent en poids, d'une manière particulièrement préférée de 0,5 à 10 % en poids, en particulier de 0,5 à 5 % en poids.

3. Procédé selon l'une des revendications précédentes, **caractérisé en ce que** les ozonures organiques sont fabriqués par traitement d'acide oléique avec de l'oxygène sec contenant de l'ozone.

4. Procédé selon l'une des revendications précédentes, **caractérisé en ce que**, lors de la fabrication des ozonures organiques et des hydroperoxydes organiques, on transforme en les ozonures organiques plus de 60 % en poids, de préférence plus de 80 % en poids, en particulier plus de 90 % en poids des acides gras insaturés.

5. Procédé selon l'une des revendications précédentes, **caractérisé en ce que** les ozonures organiques et les hydroperoxydes organiques sont, avant leur utilisation sur le matériau, stockés dans un emballage étanche à la diffusion de l'eau.

6. Procédé selon l'une des revendications précédentes, dans lequel, pour la fabrication du mélange destiné à la pulvérisation, on ajoute aux ozonures organiques et aux hydroperoxydes organiques de l'eau qui a été portée à l'équilibre de température dans la plage de 20 °C à 50 °C, en particulier de 30 °C à 40 °C, d'une manière particulièrement préférée d'environ 30 °C.

7. Procédé selon l'une des revendications précédentes, dans lequel, pour la fabrication du mélange destiné à la pulvérisation, on ajoute de l'eau aux ozonures organiques et aux hydroperoxydes organiques, et on chauffe le mélange obtenu à une température entre 20 °C et 50 °C, de préférence à environ 30 °C.

8. Procédé selon l'une des revendications précédentes, dans lequel la pulvérisation sur le matériau a lieu à l'aide d'une buse, dans laquelle le mélange est, sous pression, mélangé à un fluide secondaire, en particulier de l'air ou un liquide tel que l'eau.

9. Procédé selon l'une des revendications précédentes, **caractérisé en ce que** le procédé est mis en œuvre à l'air libre ou dans un espace pour l'essentiel fermé.

10. Procédé selon l'une des revendications précédentes, **caractérisé en ce que** le matériau est placé dans un récipient, et que le procédé est mis en œuvre dans le récipient.

11. Procédé selon l'une des revendications précédentes, **caractérisé en ce que** le matériau comprend des plantes, le procédé étant utilisé pour la lutte contre les ravageurs des plantes, les plantes étant des plantes ligneuses ou des vignes, ou des greffes ou des plantes greffées.

12. Procédé selon l'une des revendications 1 à 10, **caractérisé en ce que** le matériau comprend des déchets, le procédé étant utilisé pour le traitement désinfectant des déchets.
